# EUROPEAN PATENT SPECIFICATION

(11) **EP 2 115 922 B1**
(45) Date of publication and mention of the grant of the patent: **15.10.2014**
(21) Application number: 07709299.7
(22) Date of filing: 31.01.2007
(51) Int. Cl.: H04L 1/00, H04L 12/28, A61N 1/08, A61N 1/372

(54) **METHOD FOR SEGMENTATION IN A MEDICAL TELEMETRY SYSTEM**
VERFAHREN ZUR SEGMENTIERUNG IN EINEM MEDIZINISCHEN TELEMETRIESYSTEM
PROCÉDÉ POUR UNE SEGMENTATION DANS UN SYSTÈME DE TÉLÉMÉTRIE MÉDICALE

(43) Date of publication of application: 11.11.2009
(73) Proprietor: St. Jude Medical AB, 175 84 Järfälla (SE)
(72) Inventor: KANTERVIK, Ingemar, S-147 30 Tumba (SE)
(74) Representative: Sjölander, Henrik
(86) International application number: PCT/SE2007/000083
(87) International publication number: WO 2008/094080

(56) References cited:
- WO-A1-2006/107244
- US-A1- 2004 042 440
- US-A1- 2004 088 027
- US-A1- 2005 111 416
- "Specification of the Bluetooth System - Version 2.0 + EDR" 4 November 2004 (2004-11-04), XP002579899 Retrieved from the Internet: URL:http://bluetooth.com/Specification%20D ocuments/Core_v210_EDR.zip> [retrieved on 2010-04-27]
- "Electromagnetic compatibility and Radio spectrum Matters (ERM); Short Range Devices (SRD); Ultra Low Power Active Medical Implants (ULP-AMI) and Peripherals (ULP-AMI-P) operating in the frequency range 402 MHz to 405 MHz; Part 1: Technical characteristics and test methods; Draft ETSI EN 301 839-1" ETSI STANDARDS, LIS, SOPHIA ANTIPOLIS CEDEX, FRANCE, vol. ERM-RP08, no. V1.2.1, 1 May 2006 (2006-05-01), XP014034052 ISSN: 0000-0001
- "Electromagnetic compatibility and Radio spectrum Matters (ERM); Short Range Devices (SRD); Ultra Low Power Active Medical Implants (ULP-AMI) and Peripherals (ULP-AMI-P) operating in the frequency range 402 MHz to 405 MHz; Part 2: Harmonized EN covering essential requirements of article 3.2 of the R&" ETSI STANDARDS, LIS, SOPHIA ANTIPOLIS CEDEX, FRANCE, vol. ERM-RP08, no. V1.2.1, 1 May 2006 (2006-05-01), XP014034053 ISSN: 0000-0001
- 'Data Integrity in 802.16.4 MAC' IEEE 802.16 BROADBAND WIRELESS ACCESS WORKING GROUP; IEEE 802.16.4-01/06, [Online] 13 January 2001, XP003018647 Retrieved from the Internet: <URL:http://www.ieee802.org/16/tg4/contrib/ 802164c-01_06.pdf>
- BRADLEY P D ED - SODAGAR A M ET AL: "An ultra low power, high performance Medical Implant Communication System (MICS) transceiver for implantable devices", BIOMEDICAL CIRCUITS AND SYSTEMS CONFERENCE, 2006. BIOCAS 2006. IEEE, IEEE, PISCATAWAY, NJ, USA, 29 November 2006 (2006-11-29), pages 158-161, XP031307219, ISBN: 978-1-4244-0436-0

## Description

### Field of the invention

The present invention is related to the field of medical telemetry, and in particular to a method for segmentation and reassembly of data transmitted in a medical telemetry system.

### Background of the invention

Telemetry is a generic term for techniques for conveying measuring data from one point to another, usually by means of radio or cable connections. Within the medical field, telemetry systems are generally used for enabling radiofrequency (RF) communication between a device worn by a patient, for example an implantable medical device such as a pace maker, and an external monitoring device.

The communication of messages between the implantable medical device and the external monitoring device should be made as efficient and as secure as possible. In medical telemetry applications real-time physiologic data is transmitted. It is therefore most important that as few communication messages as possible are lost or delayed.

However, the nature of air interface means that some bit errors will inevitably be introduced from time to time, and it is important to minimize the effects of lost individual bits or lost frames. One way to alleviate this is to apply error detection and error correction schemes.

If the communication between the implantable medical device and the external monitoring device is deficient, then an excessive number of retransmissions is required, which increases the power consumption of the implantable medical device. Besides the risk of losing real-time physiological data, the service time of the battery of the IMD is also shortened.

Another aspect of rendering the communication as efficient as possible is to bundle up the data to be transmitted as efficiently as possible. Communication packets are often required to be made smaller to speed them through the network or because of specified packet size restrictions in a given path. A segmentation and reassembly process is a process to accomplish this, that is, to break a communication packet into smaller units before transmission and reassembling them into the proper order at the receiving end of the communication.

In a medical telemetry system complying with the MICS (Medical Implant Communication System) standard the data is transmitted continuously at specified instants of time regardless of the available amount of data at that time. In order for the receiving side to be able to reassemble the originally transmitted data, functionality to this end is needed. This segmentation and reassembly process should be made as efficient as possible.

Further, a design difficulty in medical telemetry systems is the limited size available in an implantable medical device for the electronics required for enabling communication.

Published patent document US 6,128,528 relates to one aspect of the way the communication is to be conducted between devices in a medical system. In particular, a method for detecting corrupt implantable medical device data within a data block is disclosed. In accordance with the disclosed invention, an error code is calculated and appended to a block or blocks of implantable medical device data as the implantable medical device data are moved and/or read by a block mover/reader in a block move or read operation within an implantable medical device.

Thus, it is realized that a number of considerations have to be made when designing a medical telemetry system, and in view of this it would be desirable to provide improvements in such a system.

US 2004/0088027 A1 discloses an implantable device that collects and aggregates data from non-implanted medical devices external from a patient body. The implantable device includes a wireless transceiver to acquire physiological data from the external medical devices, and a storage medium to store the physiological data. A processor retrieves the physiological data and communicates the physiological data to a remote patient management system.

Bradley, An Ultra Low Power, High Performance Medical Implant Communication System (MICS) Transceiver for Implantable Devices, Biomedical Circuits and Systems Conference, 2006, BIOCAS 2006, IEEE, Piscataway, NJ, USA, 29 November 2006, pag4es 158-161 discloses a 402-405 MHz MICS band transceiver for implantable medical applications. The transceiver offers low power consumption and high data rate. The circuit features an ultra low power wakeup system.

### Summary of the invention

It is a general object of the invention to provide improvements in the communication between devices of a medical telemetry system.

It is another object of the invention to provide an improved segmentation and reassembly functionality within a medical telemetry system. In particular, it is an object to provide a method in a medical telemetry system that renders the segmentation and reassembly efficient and secure.

It is another object of the invention to provide a high reliability in data transmission and in system operation.

These objects, among others, are achieved by a method, by an implantable medical device and by an external monitoring device as claimed in the independent claims.

In accordance with the invention, a method in a medical telemetry system is provided for communication between an external monitoring device and an implantable medical device. The method comprises a first step of segmenting, at a transmitting end, an implantable medical device frame layer packet into one or more Medical Implant Communication System, MICS, data blocks of a MICS radio packet. In a second step of the method a start of the implantable medical device frame layer packet is indicated, at said transmitting end, by including in a first MICS radio packet data block a segmentation and reassembly indicator having a first value. Thereafter, the MICS radio packet data blocks are transmitted, at the transmitting end and to a receiving end, over a short-range MICS radio link. Finally, theMICS radio packet data blocks are reassembled, at the receiving end into the originally transmitted implantable medical device frame layer packet. By providing such SAR functionality an efficient method for conveying radio packets is provided. The communication between the implantable medical device and the external monitoring device is rendered secure and efficient.

The method further comprises the step of including, at said transmitting end, the segmentation and reassembly indicator in MICS radio packet data blocks following the first MICS radio packet data block, but now having a second, different value. The second value indicates that the originally sent implantable medical device frame layer packet is continued in this data block. It is possible to segment the implantable medical device frame layer packet into one or into several radio packet data blocks.

The method further comprises the step of including cyclic redundancy check bits in the MICS radio packet. Increasing the length of the CRC checksum will result in fewer bit errors passing unnoticed through the MICS link, It is possible to detect errors at the radio layer, whereby the power consumption of the implantable medical device can be lowered and its service life prolonged. Further, detecting errors at this lower layer will also be time saving, since retransmissions can be handled at the radio link level.

In accordance with an embodiment of the invention, one segmentation and reassembly bit is included for each data block of the radio packet, wherein each data block comprises 14 bytes. This means little overhead and the use of the available data bandwidth is maximized in that as much payload as possible can be conveyed.

In accordance with another embodiment of the invention, the segmentation and reassembly indicator may comprise a single bit, the indicator then taking the values 1 and 0, respectively for indicating the start or continuation of an implantable medical device frame layer packet. Alternatively, the segmentation and reassembly indicator may comprise two bits, whereby it is possible to indicate the start of an implantable medical device frame layer packet, continuation and end of it. Design flexibility is thereby provided, enabling adapting the method in dependence on specific needs.

The invention is also related to a medical implantable device and a base station comprising means for performing the method, whereby advantages similar to the above are achieved.

Further characteristics of the invention and advantages thereof will be evident from the following description and the accompanying figures.

### Brief description of the drawings

Figure 1 is a schematic overview of a medical telemetry system in which the present invention may be utilized.
Figure 2 is an overview over frame layer protocols used for communication between devices of a medical telemetry system.
Figure 3 illustrates the implantable medical device layer formats.
Figure 4 illustrates a basic radio packet.
Figure 5 illustrates a data block.
Figure 6 illustrates the byte that conveys the SAR bit.
Figure 7 illustrates a layer-to-layer relationship.
Figure 8 illustrates another layer-to-layer relationship.
Figure 9 illustrates an IMD frame divided into several radio packets.
Figures 10a and 10b, respectively, illustrate the minimum and maximum size of an IMD frame.
Figure 11 is a flowchart over steps included in a method in accordance with the present invention.

### Detailed description of embodiments

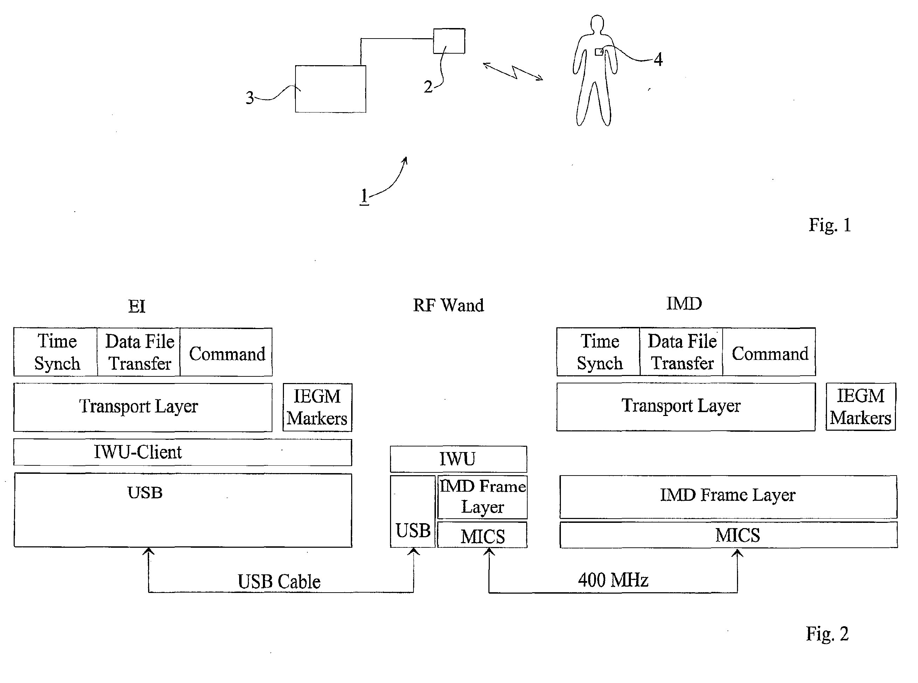

Figure 1 illustrates schematically a medical telemetry system 1 in which the present invention may be implemented. The medical telemetry system 1 comprises a base station, also denoted wand 2, which typically communicates with an external instrument, such as an external programmer or monitoring device 3 through a wired connection (e.g. a USB connection). The wand 2 and the external monitoring device 3 may be an integrated unit or two separate units. The wand 2 communicates wirelessly with one or more implantable medical device (IMD) 4, such as for example a pace maker or an internal cardiac defibrillator.

In the following description, curled braces, { }, are used for denoting messages that are exchanged between peer entities (e.g. {Stream Data Frame}), and square brackets, < >, are used for denoting elements (fields) of a frame (e.g. <Sequence Number>). Further, a radio packet comprises data blocks and is used for denoting the lowest physical layer providing the means for transmitting raw bits. That is, the physical layer performs services requested by the IMD frame layer.

Figure 2 is an overview over a protocol suite used for the communication between devices of the medical telemetry system 1. In particular, the protocol suites for the IMD 4, for the RF wand 2 (in the MICS standard known as "ultralow power active medical implant periphery", ULP AMI-P) and for the external monitoring device 3 are shown in the figure.

The present invention is primarily related to and concerned with an IMD frame layer protocol. The IMD frame layer protocol of the protocol suite is run on top of the MICS radio physical layer. The IMD frame layer protocol is used for effectuating the wireless communication between the RF Wand and the implantable medical device. The purpose of the IMD frame layer is to provide services for multiple access points of connectionless message transfer for passing information across the radio link.

Segmentation and reassembly (SAR) refers to the process used to fragment and reassemble data packets so as to allow them to be transported across the desired communication link. A MICS channel can transport 14 bytes of payload, that is, 112 bits of information. If the IMD frame layer protocol uses packets of a larger size, and this will most likely be the case, then the transmitting side, in the following example the wand 2 (which receives the data to be transmitted from the external monitoring device 3), has to divide, i.e. segment, the packets into a stream of smaller packets. That is, the large communication packet (i.e. the IMD frame layer packet) has to be segmented into chunks of 14 bytes, which can be sent over the MICS link. The receiver then has to put together, i.e. reassemble, the received 14 bytes chunks into the originally sent IMD frame layer packet.

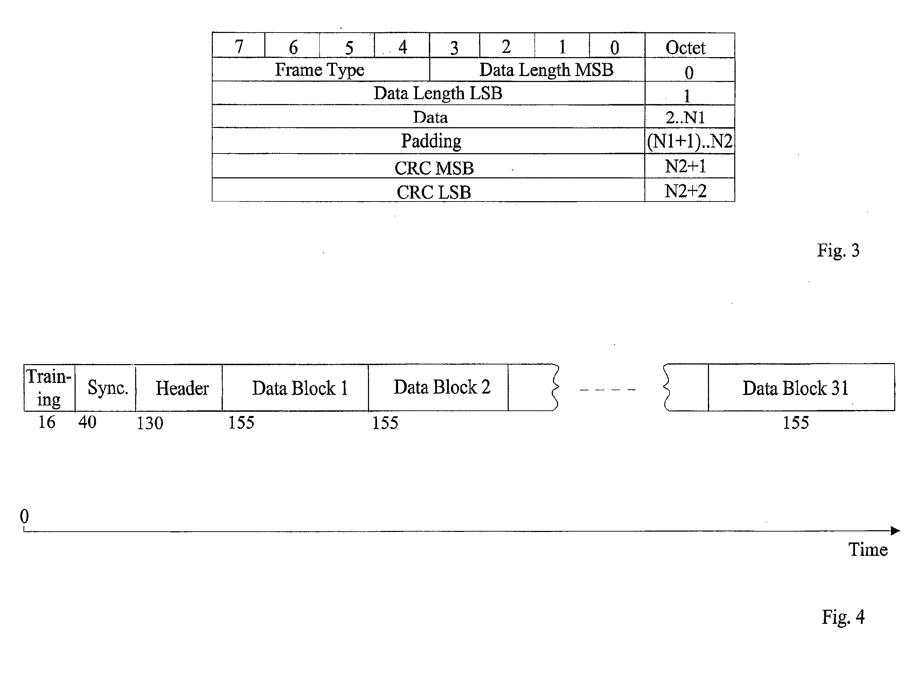

Figure 3 illustrates the IMD frame layer format. Frame Type identifies the current IMD frame type. There are several IMD frame types:
Packet Data Frame, carrying e.g data for programming a parameter. A Packet Data Frame conveys Transport Layer messages (the data field of the Packet Data Frame carries the transport layer messages). A Packet Data Frame is identified by setting <Frame Type> to 0 (0 0 0 0).
Stream Data Frame, carrying e.g. real-time data or markers (intracardiac electrogram markers). A Stream Data Frame is identified by setting <Frame Type> to 1 (0001).
Keep-alive Request; A Keep-alive Request is identified by setting <Frame Type> to 2 (0 0 1 0). This frame is initiated by a Keep Alive Procedure, which is a mechanism intended for detecting a temporary loss of the MICS radio link, i.e. the physical channel, and provides link quality parameters to a Link Quality Assessment Procedure. The Link Quality Assessment Procedure in turn has the purpose to assess the current link quality by means of the parameters in the wand 2 and the IMD 4. The link quality is indicated to upper layer upon a value change.
Keep-alive Reply; A Keep-alive Reply is identified by setting <Frame Type> to 3 (0 0 1 1) . This frame is sent from the IMD in response to a Keep-alive Request and contributes to the above-described Keep Alive Procedure. This message carries, for example, data related to the RF link quality.
Loopback frame; A Loopback frame is identified by setting <Frame Type> to 0xF and is used for sending data to the IMD, whereby the IMD just echoes the data back from the frame layer to the host.

The <Data Length> field is the total length of data field, wherein applicable values are, in an exemplary embodiment, from 1 to 512 bytes. In another embodiment however, the IMD frame layer is designed to carry between 1 and 4095 bytes. It is to be noted that the IMD frame layer may be designed to carry even more data. In the illustrated case, the <Data Length> field is defined by 9 bits of the available 12 bits, but if the <Data Length> field were to be defined by, for example, 12 bits, then a length of 4095 bytes would be the highest number of bytes that can be expressed. In yet other implementations, the IMD frame layer may be designed to carry even further data.

The <Data> field represents different messages as defined above. A message is governed by the content of the <Frame Type> field.

The <Padding> field is used to accommodate an IMD Frame to 14 bytes alignment, which, as mentioned earlier, is required for MICS radio packet data blocks. This field contains undefined octet values and may be of any size between 0 and 12 bytes. The padding field is included in the cyclic redundancy check algorithm. The CRC algorithm is used by the IMD frame layer and is sent in the last data block. The CRC algorithm may for example be a CRC-16 polynomial. An exemplary polynomial used for the CRC algorithm is G(x) = x¹⁶ + x¹² + x⁵ + 1. Other polynomials may be used.

Figure 4 illustrates a basic MICS radio packet, onto which the IMD frames described above are to be mapped. The radio packet may carry up to 31 data blocks, each data block able to convey 113 bits of user data. In particular, each data block conveys a payload of 14 bytes plus one extra bit denoted a SAR (Segmentation and Reassembly) bit. In the figure a 16 bits training sequence, 40 bits for synchronisation and a header comprising 140 bits are shown as an example.

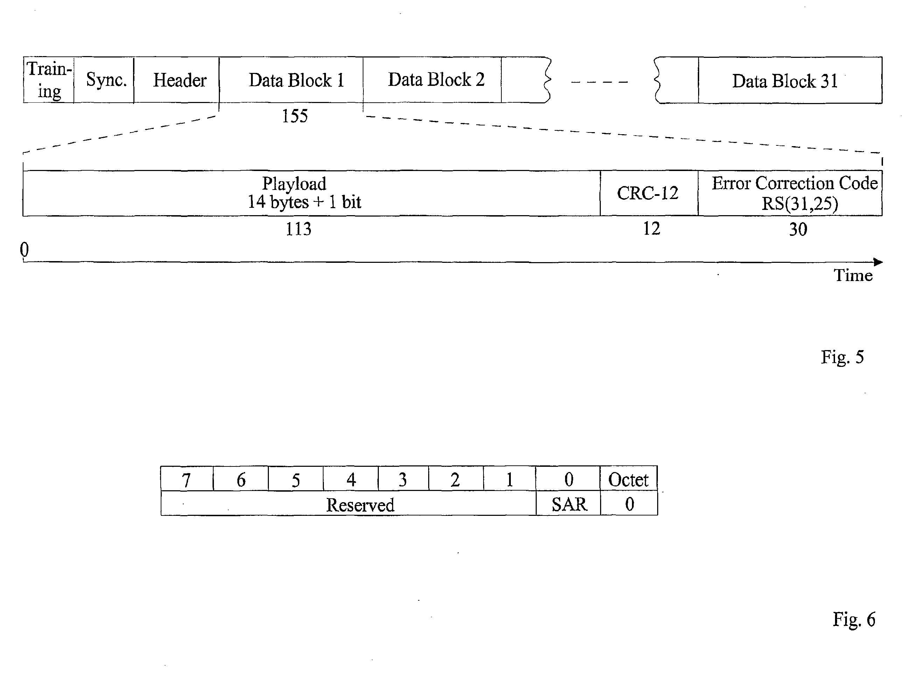

Figure 5 illustrates a data block of a radio packet more in detail. The data block comprises 14 bytes of payload, i.e. user information, and a SAR bit. Further, a cyclic redundancy check (CRC) comprising for example 12 bits is included, i.e. a conventional checksum computed and appended before transmission, and verified afterwards by the receiving side to confirm that no changes occurred during transmission. Further still, conventional error correction code comprising for example 30 bits is also included. As an example, Reed-Solomon error correction code could be utilized, e.g. RS(31,25).

The SAR bit could be used to indicate whether user data within a data block is a fragment or not. However, in accordance with the invention, the SAR bit is used to indicate the start of an IMD frame or continuation of the IMD frame. The SAR bit is represented by a byte and could have the value zero (0) or one (1). Figure 6 illustrates the byte that conveys the SAR bit. The SAR bit indicating the start of the IMD frame can have the value zero ("0") or one ("1"). A continued data block is then indicated by the other value. In an alternative embodiment, the segmentation and reassembly indicator comprises two or more bits. The segmentation and reassembly indicator may then have several values, e.g 00, 01, 10, 11. It is thereby possible to indicate the start, the end and even the middle of the IMD frame.

The SAR bit is represented as a byte when it is delivered to/from the MICS layer and a SAR bit is sent for each data block. That is, every 15^{th} byte to/from the MICS layer is a byte that carries the SAR bit. The SAR byte is included in the CRC computation as well as the <Frame Type> field, the <Data Length> field, the <Data> field and the <Padding> field.

Only 1 SAR bit is needed for each data block, that is, just 1 SAR bit per 14 bytes. The SAR functionality of the invention thus adds little overhead data and the available data bandwidth is thereby maximized.

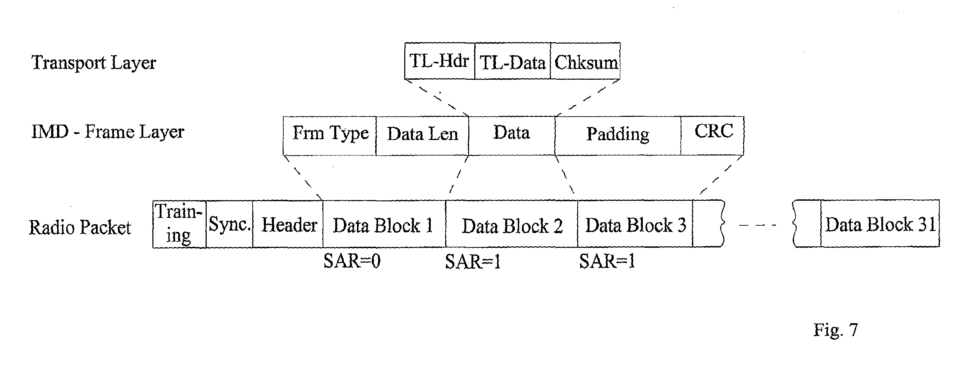

Figure 7 illustrates the relationship of the messages, illustrated from layer to layer starting at Transport Layer (on the top). The transport layer data (e.g. for maintaining flow control of data and recovery of data between devices) are mapped onto IMD frame packets, as is illustrated. Transport layer messages are carried by the <Data> field of the IMD frames.

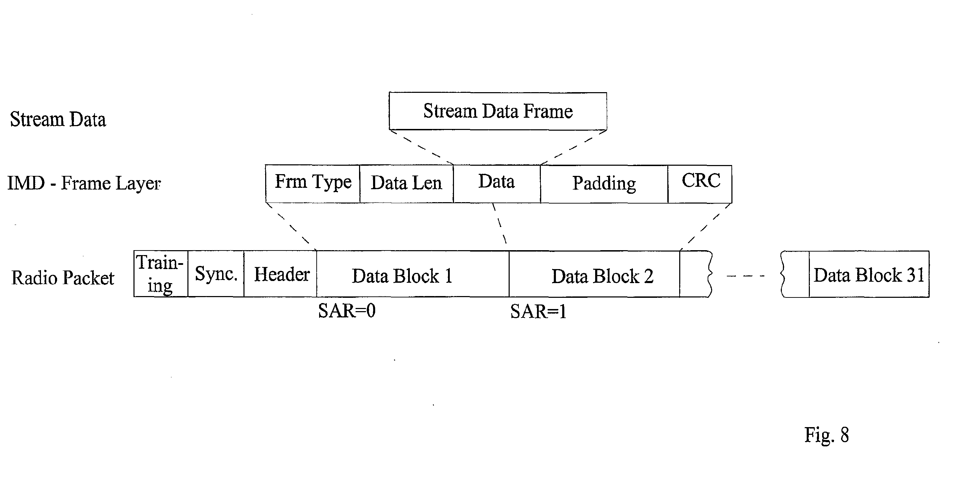

Figure 8 illustrates the relationship of the messages from layer to layer, starting at Stream Data Layer. In this example, <Frame Type> is set to 1 (0 0 0 1), i.e. identifying stream data, the <Data Length> is set to whatever length the <Data> field has (between 1 and 512 bytes), followed by data, padding bits and CRC bits, as described above. The IMD frame is mapped onto the radio packet in chunks of 14 bytes plus 1 SAR bit. In a preferred embodiment, the start of the IMD frame is indicated by setting the SAR bit of the first radio packet data block to 0 (zero). The subsequent chunks of data (contained in 14 bytes plus 1 SAR bit) are indicated by a 1 (one), i.e. the respective SAR bits of the following data blocks are set to 1 (one) for indicating that the data block is a continuation of the IMD frame.

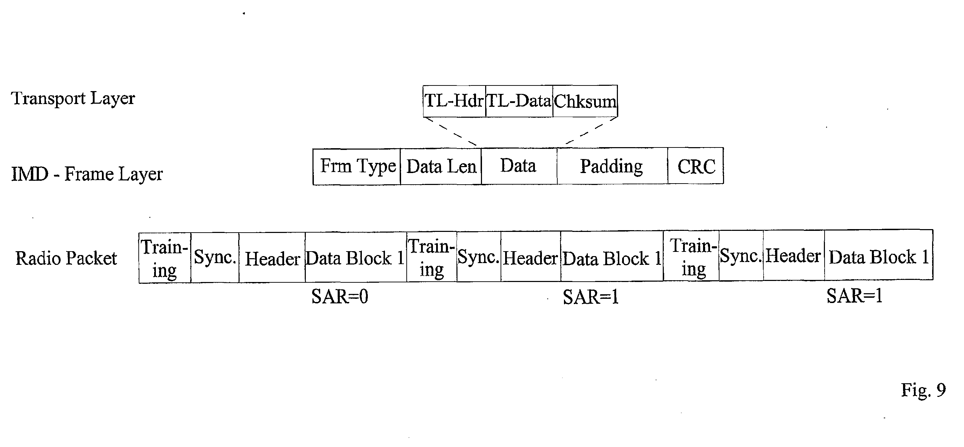

If the IMD frame contains more information than what can be fitted into a radio packet, then the IMD frame is suitably divided. The IMD frame may be divided into several radio packets, which is illustrated in figure 9. The IMD frame layer cannot control whether an IMD frame should be carried by one radio packet or not, this is governed by the MICS layer only. That is, the MICS chip or the RF chip, i.e. the radio module of the wand 2 and the IMD 4, transmits data with certain time intervals and whatever data is within the data buffer of the MICS chip at this time is mapped onto a radio packet and transmitted. This means that irrespective of whether or not more data would fit into a radio packet at the time of transmission, the radio packet is transmitted. In the illustrated example, the IMD frame is divided into several radio packets, and the first data block of the first radio packet has a SAR bit 0, the first data block of the second radio packet is a continuation of the IMD frame and has SAR bit 1, the first data block of the third radio packet is also a continuation of the IMD frame and thus has SAR bit 1 and so on.

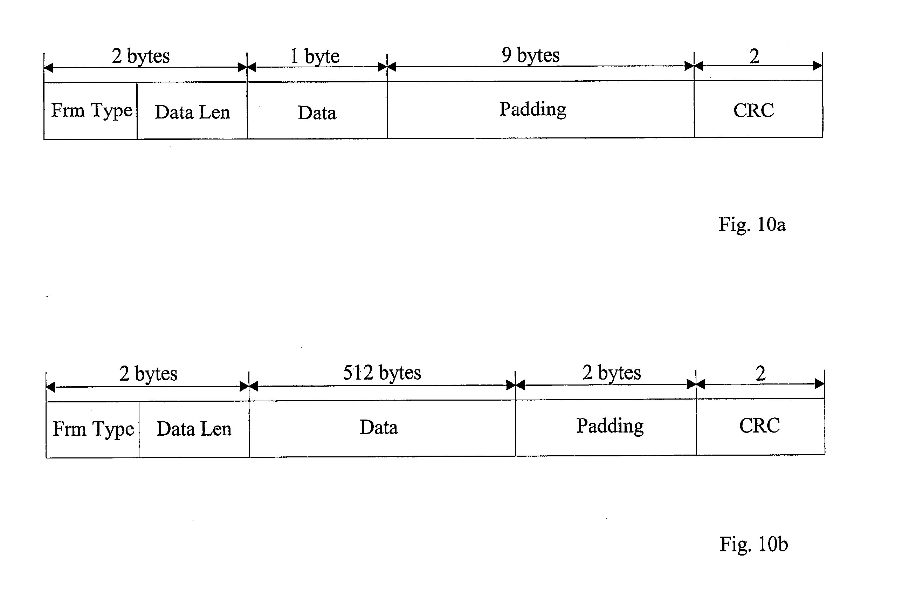

The total length of an IMD frame is derived through the <Data Length> field (described earlier) and the fact that a frame has to be aligned with a 14 bytes data block carried by a radio packet. Figure 10a shows an IMD frame that carries a <Data> field with minimum <Data Length>, which is 1 byte. A padding of 9 bytes is then needed in order to be aligned with a data block of a radio packet. The frame type field, the data length field and the CRC field require in total 4 bytes and the frame length is thus 14 bytes, which is equal to one data block. It is noted that the <Data Length> field is equal to 1 for a minimum size of an IMD Frame, although it's total frame length is 14 bytes.

Figure 10b shows an IMD frame that carries a <Data> field with maximum <Data Length>, which is, in the illustrated case, 512 bytes. A padding of 2 bytes is needed in order to be aligned with the data blocks. This gives a maximum frame length of 518 bytes, which is equivalent to 37 (= 518/14) radio frame data blocks. It is noted that the <Data Length> field is equal to 512 for a maximum size of an IMD Frame, although its total frame length is 518 bytes.

### Procedure during transmission of IMD Frame

The following procedure is used during transmission of an IMD frame at the IMD frame layer.

In the typical case, a request to transmit a Packet Data is received from an upper layer. A frame fragment of 14 bytes is then created, wherein the frame fragment fits into a radio packet data block. The frame fragment is assigned a <Frame Type> field and its value is set to Packet Data. The frame fragment is also assigned a <Data Length> field and the value is equal to the length of the <Data> field of the Packet Data. At least 1 and up to 12 bytes are added from the upper layer data to the frame fragment.

If the current frame fragment is the last fragment, which will be the case when the upper layer requests to send between 1 and 10 bytes, then an appropriate number of zeros (between 0 and 9 bytes) are added for padding. CRC bytes are also added (2 bytes). The frame fragment is sent to the MICS layer by means of a request that sets SAR bit to value "First block".

If the current frame fragment is not the last fragment then a new frame fragment is created (14 bytes) and 1-14 bytes are added from the upper layer data to this frame fragment.. The frame fragment is sent to the MICS layer by means of a request that sets SAR bit to value "Subsequent block". This is repeated while new frame fragments are needed, i.e. until a last frame fragment is created, then 0-12 bytes are added for padding and also CRC bytes (2 bytes).

In a request at the IMD side, the steps are as above, except that the request from upper layer is to transmit Stream Data, and the <Frame Type> is accordingly set to Stream Data. Further, an internal byte counter is copied to a <Sequence Number> field and the counter is incremented by one for each radio packet transmitted. This counter is initialized to zero when a MICS radio link has been established.

### Procedure during reception of IMD Frame

The following procedure is used during reception of an IMD frame at the IMD frame layer.

The IMD frame layer receives radio packet data blocks and a SAR bit from the MICS radio layer (see for example figure 2 and 7). As described, the SAR bit indicates the beginning of a new IMD frame and several radio packet data blocks may be assembled to form a complete IMD frame. In the typical case, a radio packet data block is received and the SAR bit indicates a "First block". It is verified that the <Frame Type> field is valid and that the <Data Length> field is valid (i.e. a value greater than zero and lesser than 513). If more radio packet data blocks are expected then a new radio packet data block is awaited, and it is verified that the SAR bit indicates "subsequent block" (or equivalently "continued frame"). The frame is concatenated with the previously received frame. Thereafter it is verified that a correct <CRC> field is received. Finally the payload is extracted from the IMD frame and indicated to the upper layer. If the current frame is a stream data frame then this frame is further conveyed to Receive Stream Data Procedure (described next).

### Receive Stream Data Procedure

This procedure is run only in the RF wand 2 and it is run on top of the receive IMD frame procedure (described earlier). This procedure is started as soon as a radio link has been established between the RF wand 2 and the IMD 4. The procedure has two active states: *Wait for Synch* and *Is Synch.* A {Stream Data Frame} message that is received in *Wait for Synch* state will be discarded. If a {Stream Data Frame} message is received in *Is Synch* state, the message payload will be extracted and delivered by means of an indication to upper layer. The latter state opens the stream data flow between the IMD 4 and the external monitoring device 2.

A {Stream Data Frame} message carries a <Sequence Number>, which number is cleared (in the IMD 4) when a MICS radio link has been established. The RF wand 2 verifies that the <Sequence Number> incremented properly, i.e. increased by one for every new message. Each {Stream Data Frame} message is time stamped upon reception by the RF wand 2. This enables the RF wand 2 to measure the time interval between two consecutive {Stream Data Frame} messages.

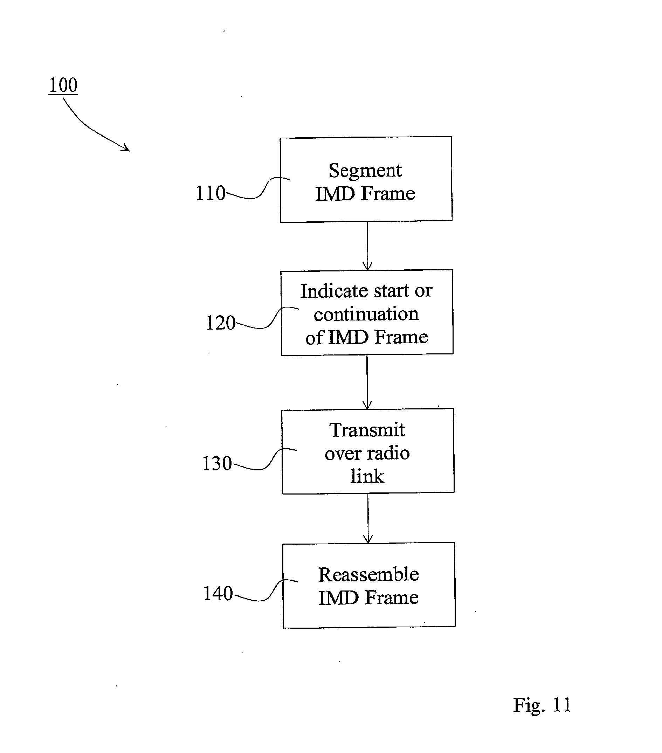

Figure 11 illustrates the steps of the method in accordance with the present invention and also summarizes some central aspects of the present invention. The method 100 comprises a first step, step 110 of segmenting, at the transmitting end, an IMD frame layer packet into one or more data blocks of one or more radio packets, which data blocks are transmitted in the communication between the wand 2 and the IMD 4. In a second step, indicated at 120, the start or continuation of the IMD frame layer packet is indicated by including in the first data block of the radio packet a segmentation and reassembly indicator (SAR bit) having a first value. The start of the IMD frame layer packet can be indicated by a single bit and the value can then be a zero ("0") or a one ("1"), as described earlier. As also described earlier, if the IMD frame layer packet comprises more data than what can be fitted in one radio packet, and this will typically be the case, then the data blocks of the radio packet following the first data block are also provided with a segmentation and reassembly indicator. These data blocks are a continuation (fragment) of the IMD frame and are indicated by a value differing from the indicator of the first data block indicating the start of the IMD frame. In a third step, indicated at 130, the data blocks are transmitted over radio interface, more specifically a short-range medical radio link, at the transmitting end. Finally, in step 140, the data blocks are reassembled at the receiving end, into the originally transmitted IMD frame layer packet.

In another, optional step of the method, cyclic redundancy check (CRC) bits are included, at a transmitting end, in the last data block of the radio packet. If, at a receiving end, a CRC error is detected, then saved data intended for the matching IMD frame layer packet is discarded. If an indication of a transmission error is received from the MICS layer, then any ongoing transmission is discarded, upon which a new transmit request (request to transmit Packet Data or request to transmit Stream Data) is awaited from the upper layer.

It is to be noted that data can be discarded in dependence on the type of data that is sent. For example, if stream data is sent, then it may happen that the data is not of any interest after a certain period of time, and therefore discarded if a transmission has failed. Packet data, on the other hand, may be retransmitted until the radio channel is lost.

If the length of the CRC checksum is increased, this will result in fewer bit errors passing unnoticed through the MICS link. That is, the errors can be found at the radio link level and the radio modules may then perform retransmissions, else such retransmissions are made at a higher level, which entails the transmission of larger data packets. Stated differently, when errors are detected, retransmissions handled on the lower layers are less costly in terms of power consumption and time, compared to being handled by the higher layers. There are thus benefits of including more CRC bits. Adding one bit for the SAR functionality can be combined with an increase of the CRC checksum length, which would result in a data block comprising 112 information bits, 1 SAR bit, and 12 CRC bits amounting to a total of 125 bits, which are then encoded using RS(31, 25).

On the radio link layer (MICS chip) a CRC-12 algorithm can be utilized on each data block provided by the MICS chip, having the following polynomial: CRC-12 = x¹² + x¹¹ + x³ + x² + x¹ + x. In the IMD frame layer any CRC algorithm may be implemented, for example the CRC-16 algorithm, that is, the earlier mentioned polynomial G(x) = x¹⁶ + x¹² + x⁵ + 1. Yet other polynomials may be used in alternative embodiments, CRC-32 being yet an example.

As mentioned in the introductory part, one particular difficulty encountered in medical telemetry systems is the limited size available in the implantable medical device for the electronics required for enabling communication. In the above-described invention, this can be taken into account. More specifically, the available space in the IMD for, for example, needed memory means (e.g. buffer for storing data to be transmitted) has to be considered when determining the amount of data to transmit. Further, if the packet data packets are allowed to be too large, then there will be scheduling issues when a user wishes to transmit streaming data. If the stream data packets are allowed to be too large, then there may be a too large delay before the data received is actually displayed to a physician monitoring for example the heart activity of a patient. The invention provides a flexible solution, wherein the different aspects, such as device sizes, delay and so on, can be balanced and taken into account. The solution most appropriate for a particular application can then be chosen. For example, an exemplary size of stream data frame size is 5 radio data blocks, which equals 5 x 14 = 70 bytes. This frame may contain six samples of Intra cardiac electrogram (IEGM) data and optional markers. The delay before the physician actually sees the data is then still acceptable.

The invention is also related to a medical telemetry system 1 comprising means for performing the above methods. In particular, the medical telemetry system 1 comprises a transmitting end, e.g. the RF wand 2 or the implantable medical device 4, and a receiving end, again, either the RF wand 2 or the implantable medical device 4. The transmitting and receiving sides comprise radio modules (MICS chips) and means for implementing the methods described above. The methods may be implemented in hardware and/or software.

While the present invention has been described in various embodiments it shall be appreciated that the invention is not limited to the specific features and details set forth, but is defined only by the appended patent claims.

## Claims

1. A method in a medical telemetry system for communication between an external monitoring device (3) and an implantable medical device (4) of said medical telemetry system (1), said method comprising the steps of:
segmenting (110), at a transmitting end, an implantable medical device frame layer packet into one or more Medical Implant Communication System, MICS, radio packet data blocks of a MICS radio packet,
indicating (120), at said transmitting end, a start of said implantable medical device frame layer packet by including in a first MICS radio packet data block a segmentation and reassembly indicator having a first value,
including, at said transmitting end, in MICS radio packet data blocks following said first MICS radio packet data block said segmentation and reassembly indicator having a second, different value for indicating a continuation of said implantable medical device frame layer packet,
including cyclic redundancy check, CRC, bits in said MICS radio packet,
transmitting (130), at said transmitting end and to a receiving end, said MICS radio packet data blocks over a short-range MICS radio link, and
reassembling (140), at said receiving end, said MICS radio packet data blocks into the originally transmitted implantable medical device frame layer packet.

2. Method as claimed in claim 1, wherein one segmentation and reassembly bit is included for each MICS radio packet data block of said MICS radio packet, each MICS radio packet data block comprising 14 bytes.

3. The method as claimed in claim 1 or 2, wherein said segmentation and reassembly indicator comprises a single bit.

4. The method as claimed in claim 3, wherein said first value is one of 1 and 0, and said second, different value is the other of 1 or 0.

5. The method as claimed in claim 1 or 2, wherein said segmentation and reassembly indicator comprises two bits.

6. The method as claimed in claim 5, wherein said segmentation and reassembly indicator comprises values for indicating the start of an implantable medical device frame layer packet, continued implantable medical device frame layer packet and end of implantable medical device frame layer packet.

7. A medical implantable device (4) **characterized by** means for performing a method as claimed in any of claims 1-6.

8. A medical monitoring device (3) **characterized by** means for performing a method as claimed in any of claims 1-6.

## Patentansprüche

1. Verfahren in einem medizinischen Telemetriesystem für den Datenaustausch zwischen einer externen Überwachungseinrichtung (3) und einem implantierbaren Medizinprodukt (4) des medizinischen Telemetriesystems (1), wobei das Verfahren folgende Schritte umfasst:
Segmentieren (110), auf einer Sendeseite, eines Frame-Schicht-Pakets des implantierbaren Medizinprodukts in ein oder mehrere Funkpaketdatenblöcke nach dem Kommunikationssystem für medizinische Implantate, MICS, eines MICS-Funkpakets,
Angeben (120), auf der Sendeseite, eines Anfangs des Frame-Schicht-Pakets des implantierbaren Medizinprodukts durch Aufnehmen eines Segmentierungs- und Wiederzusammensetzungsanzeigers mit einem ersten Wert in einen ersten MICS-Funkpaketdatenblock,
Aufnehmen, auf der Sendeseite, in MICS-Funkpaketdatenblöcken nach dem ersten MICS-Funkpaketdatenblock des Segmentierungs- und Wiederzusammensetzungsanzeigers mit einem zweiten, anderen Wert zum Angeben einer Fortsetzung des Frame-Schicht-Pakets des implantierbaren Medizinprodukts,
Aufnehmen von Bits für die zyklische Redundanzprüfung, CRC, in dem MICS-Funkpaket,
Senden (130), auf der Sendeseite und an eine Empfangsseite, der MICS-Funkpaketdatenblöcke über eine Kurzstrecken-MICS-Funkverbindung, und
erneutes Zusammensetzen (140), auf der Empfangsseite, der MICS-Funkpaketdatenblöcke zu dem ursprünglich gesendeten Frame-Schicht-Paket des implantierbaren Medizinprodukts.

2. Verfahren nach Anspruch 1, wobei ein Segmentierungs- und Wiederzusammensetzungsbit für jeden MICS-Funkpaketdatenblock des MICS-Funkpakets aufgenommen wird, wobei jeder MICS-Funkpaketdatenblock 14 Bytes umfasst.

3. Verfahren nach Anspruch 1 oder 2, wobei der Segmentierungs- und Wiederzusammensetzungsanzeiger ein einzelnes Bit umfasst.

4. Verfahren nach Anspruch 3, wobei der erste Wert 1 oder 0 ist und der zweite, andere Wert der jeweils andere Wert, 1 oder 0, ist.

5. Verfahren nach Anspruch 1 oder 2, wobei der Segmentierungs- und Wiederzusammensetzungsanzeiger zwei Bits umfasst.

6. Verfahren nach Anspruch 5, wobei der Segmentierungs- und Wiederzusammensetzungsanzeiger Werte zum Angeben des Beginns eines Frame-Schicht-Pakets des implantierbaren Medizinprodukts, eines fortgesetzten Frame-Schicht-Pakets des implantierbaren Medizinprodukts und des Endes eines Frame-Schicht-Pakets des implantierbaren Medizinprodukts umfasst.

7. Implantierbares Medizinprodukt (4), **gekennzeichnet durch** Mittel zum Durchführen eines Verfahrens nach einem der Ansprüche 1 bis 6.

8. Medizinische Überwachungseinrichtung (3), **gekennzeichnet durch** Mittel zum Durchführen eines Verfahrens nach einem der Ansprüche 1 bis 6.

## Revendications

1. Procédé dans un système de télémétrie médicale pour la communication entre un dispositif de surveillance extérieur (3) et un dispositif médical implantable (4) dudit système de télémétrie médicale (1), ledit procédé comprenant les étapes consistant à :
segmenter (110), sur une extrémité d'émission, un paquet de niveau trame de dispositif médical implantable en un ou plusieurs blocs de données de paquet radio de système de communication d'implants médicaux, MICS, d'un paquet radio MICS,
indiquer (120), sur ladite extrémité d'émission, un début dudit paquet de niveau trame de dispositif médical implantable en incluant un indicateur de segmentation et réassemblage présentant une première valeur dans un premier bloc de données de paquet radio MICS,
inclure, sur ladite extrémité d'émission, dans des blocs de données de paquet radio MICS suivant ledit premier bloc de données de paquet radio MICS, ledit indicateur de segmentation et réassemblage présentant une seconde valeur différente pour indiquer une continuation dudit paquet de niveau trame de dispositif médical implantable,
inclure des bits de contrôle par redondance cyclique, CRC, dans ledit paquet radio MICS,
émettre (130), sur ladite extrémité d'émission et vers une extrémité de réception, lesdits blocs de données de paquet radio MICS via une liaison radio MICS à courte portée, et
réassembler (140), sur ladite extrémité de réception, lesdits blocs de données de paquet radio MICS pour former le paquet de niveau trame de dispositif médical implantable émis à l'origine.

2. Procédé selon la revendication 1, dans lequel un bit de segmentation et réassemblage est inclus pour chaque bloc de données de paquet radio MICS dudit paquet radio MICS, chaque bloc de données de paquet radio MICS comprenant 14 octets.

3. Procédé selon la revendication 1 ou 2, dans lequel ledit indicateur de segmentation et réassemblage comprend un seul bit.

4. Procédé selon la revendication 3, dans lequel ladite première valeur est soit 1 soit 0, et ladite seconde valeur différente est respectivement soit 0 soit 1.

5. Procédé selon la revendication 1 ou 2, dans lequel ledit indicateur de segmentation et réassemblage comprend deux bits.

6. Procédé selon la revendication 5, dans lequel ledit indicateur de segmentation et réassemblage comprend des valeurs destinées à indiquer le début d'un paquet de niveau trame de dispositif médical implantable, la suite d'un paquet de niveau trame de dispositif médical implantable et la fin d'un paquet de niveau trame de dispositif médical implantable.

7. Dispositif médical implantable (4), **caractérisé par** des moyens pour exécuter un procédé selon l'une quelconque des revendications 1 à 6.

8. Dispositif de surveillance médicale (3), **caractérisé par** des moyens pour exécuter un procédé selon l'une quelconque des revendications 1 à 6.
